# FASCICULE DE BREVET EUROPEEN

(11) **EP 0 852 152 B1**
(45) Date de publication et mention de la délivrance du brevet: **02.04.2003**
(21) Numéro de dépôt: 97410144.6
(22) Date de dépôt: 18.12.1997
(51) Int. Cl.: A61M 5/148, A61M 5/155

(54) **Dispositif d'injection de liquide médical**
Injektionsvorrichtung für medizinische Flüssigkeiten
Medical liquid injection device

(30) Priorité: 06.01.1997 FR 9700235
(43) Date de publication de la demande: 08.07.1998
(73) Titulaire: Medex, 74940 Annecy le Vieux (FR)
(72) Inventeur: Lacroix, Jean-Pierre, 74940 Annecy Le Vieux (FR)
(74) Mandataire: Gasquet, Denis

(56) Documents cités:
- EP-A- 0 676 214
- US-A- 3 199 511
- US-A- 4 332 246
- US-A- 4 673 392

## Description

La présente invention concerne un dispositif d'injection d'un liquide médical destiné au corps humain ou animal. Il est plus particulièrement relatif à un perfectionnement permettant de mettre en pression le liquide médical à injecter quand il est contenu dans une enveloppe souple.

Les récents développements de la médecine ont conduit notamment à mettre au point différents procédés d'analyse et de contrôle de l'état des patients. Parmi ces procédés il existe les analyses faites après injection d'un produit de contraste. C'est par exemple le cas pour les analyses du type angiographie qui consistent à injecter un produit iodé dans les vaisseaux, veines et artères pour opacifier l'ensemble de ceux-ci ainsi que les tissus et organes imprégnés d'opacifiant et ainsi, par radio, déterminer les anomalies éventuelles, comme les thromboses, les embolies, les compressions et autres. Il peut s'agir aussi d'utilisation de produits de contraste pour réaliser un scanner ou en angiographie traditionnelle ou numérisée, l'injection veineuse ou artérielle devant se faire sous forme de bolus à vitesses allant généralement de 0,5 à 35 ml/s. Mais il existe aussi bien d'autres types d'injection sans pour autant que ce soient des injections de liquide de contraste. C'est par exemple le cas des transfusions ou des procédés de nutrition artificielle, notamment par le sang ou par voie digestive (entérale ou parentérale).

On connaît déjà des injecteurs qui comprennent des seringues contenant le liquide à injecter dont la tête d'injection est reliée à une aiguille hypodermique ou intraveineuse ou à un cathéter par l'intermédiaire d'une canalisation qui comprend éventuellement une valve anti-retenue à seuil. La préparation des seringues actuelles est faite par remplissage et débullage à partir de flacons de différentes contenances telles que 60, 100, 200 millilitres, voire même plus, comme, par exemple, 500 millilitres. Devant l'exigence croissante des utilisateurs dans le domaine de l'hygiène et de la non contamination par les patients des produits utilisés, qui peuvent servir à plusieurs patients, il est donc souhaitable d'éviter au maximum les transvasements de flacon à seringue surtout si les seringues sont appelées à servir pour plusieurs patients. Le but étant d'éviter tout risque de contamination rétrograde. Le développement des maladies contagieuses, notamment du Sida, conduit les médecins à une exigence encore plus importante dans le domaine de l'hygiène et il est donc déterminant et très important de supprimer tout risque de contamination et donc de supprimer au maximum les transvasements de produits initialement nécessaires et ce dans un but principal d'hygiène et de sécurité. Les constructeurs ont donc imaginé des dispositifs dans lesquels le liquide médical est utilisé directement dans un conditionnement constitué par une poche souple munie d'un raccord. Ladite poche remplaçant ainsi la seringue traditionnelle qui est connectée à une canalisation reliée à un conduit d'injection tel qu'un cathéter ou une aiguille hypodermique, intraveineuse ou autre, est placée dans un boîtier comprenant le liquide moteur inerte mis sous pression.

De tels dispositifs sont, par exemple, décrits dans les demandes de brevets européens 0 343 286 et 0 676 214, dans le brevet américain 3 199 511 et dans les brevets US 4 332 246 et US 4 673 392, ainsi que dans la demande de brevet française 2 592 306. Dans tous ces dispositifs il est fait usage d'une poche souple contenant le liquide à injecter, laquelle poche est placée dans une enceinte contenant le liquide moteur qui, mis sous pression, aura pour mission de propulser le liquide contenu dans la poche. Cependant, si de tels dispositifs ont permis des progrès considérables dans le domaine de l'injection, ils présentent néanmoins des inconvénients liés à leur utilisation et à leur mode de construction, par exemple. En effet, certains dispositifs sont peu pratiques pour leur utilisateur, notamment, lors des changements de poche souple ou lorsqu'il est nécessaire d'évacuer de la poche des bulles d'air. De plus, dans d'autres appareils, la répartition de la pression sur la poche n'est pas satisfaisante et la précision du réglage des paramètres de l'injection qui dépend de la conception technique de la poche souple s'altère au fur et à mesure de leur utilisation. Par ailleurs, tout ces dispositifs antérieurs ne sont destinés que pour réaliser des perfusions et en aucun cas ne sont utilisés pour l'injection de produits de contraste.

La présente invention propose donc un nouveau dispositif simple, facile à utiliser, fiable et précis.

Ainsi, selon sa caractéristique principale, le dispositif destiné à mettre en oeuvre un procédé d'injection de liquide à usage médical du type qui consiste
- à utiliser une poche souple contenant ledit liquide médical à injecter, ladite poche étant étanche et comprenant au moins un orifice d'écoulement destiné à être connecté à une canalisation reliée à un conduit d'injection tel qu'un cathéter ou une aiguille hypodermique, intraveineuse ou autre,
- à déformer la poche souple par l'intermédiaire d'un liquide moteur inerte mis en pression de façon à transmettre cette pression au liquide médical à injecter afin de la forcer à s'écouler dans la canalisation en la plaçant dans l'enceinte interne d'un boîtier comprenant le liquide moteur inerte, puis à mettre en pression ledit liquide moteur,
- comprend un boîtier avec un plan de symétrie générale longitudinal, ledit boîtier étant constitué par un corps de boîtier comportant une ouverture supérieure située à l'extrémité supérieure dudit corps et des moyens d'obturation mobiles entre une position d'ouverture destinée à permettre l'introduction de la poche souple et une position d'obturation fermant l'enceinte, ledit corps de boîtier possédant une section transversale de forme allongée ovale, ovoïde, voire oblongue ou elliptique.

Selon une caractéristique complémentaire, le corps de boîtier est constitué par une paroi d'extrémité inférieure et par une paroi périphérique formée par au moins deux portions de paroi latérales de forme courbe

Selon un mode de réalisation, lesdites portions de parois latérales possèdent chacune une section transversale en forme de portion sensiblement de cercle, lesdites portions de cercles étant non concentriques et de rayon identique. De plus, selon ce mode de réalisation, ledit rayon de la section transversale des portions de paroi latérales est constant le long de l'axe longitudinal du corps de boîtier.

Par ailleurs, le boîtier comporte une enceinte déformable disposée à l'intérieur de l'enceinte du corps de boîtier. ladite enceinte déformable pouvant être formée de deux parois planes souples prolongée vers le haut par une paroi divergente.

Le dispositif comporte, en outre, des moyens de positionnement destinés à permettre le positionnement de l'enceinte déformable sensiblement dans le plan de symétrie longitudinale du boîtier.

Selon une variante, le dispositif comporte des moyens de mise en forme de l'enceinte déformable destinés à permettre à ladite enceinte de prendre sensiblement la forme d'une poche souple remplie avant l'introduction de la poche souple, lesdits moyens de mise en forme pouvant être constitués par une grille de guidage disposée autour de l'enceinte déformable de façon à positionner l'enceinte déformable et à la mettre dans une forme sensiblement identique à celle de la poche souple.

Selon le mode de réalisation préféré de l'invention, les moyens d'obturation comportent un orifice de passage permettant de raccorder la poche souple à la canalisation, lesdits moyens d'obturation pouvant être formés par un couvercle constitué en deux parties complémentaires mobiles l'une par rapport à l'autre.

Selon une caractéristique complémentaire, le plan de symétrie générale longitudinal du boîtier est disposé sensiblement verticalement, l'ouverture supérieure du corps de boîtier étant dirigée vers le haut de façon à permettre l'évacuation des bulles éventuelles retenues dans la poche.

Le dispositif de l'invention permet de pouvoir utiliser tout type et toute taille de poche souple, aussi bien celles de 50 millilitres que celle de 500 millilitres.

D'autres caractéristiques et avantages de l'invention se dégageront de la description qui va suivre en regard des dessins annexés qui ne sont donnés qu'à titre d'exemples non limitatifs.

Les figures 1 à 12 illustrent un mode de réalisation préféré du dispositif selon l'invention.
La figure 1 est une vue illustrant de façon schématique l'ensemble du dispositif d'injection.
Les figures 2 et 3 sont des vues montrant un exemple de poche déformable contenant le liquide médical, destinée à être utilisée dans le dispositif de l'invention ; la figure 2 étant une vue extérieure frontale, la figure 3 étant une vue latérale avec arrachement partiel.
La figure 4 est une vue en perspective du corps de boîtier et des moyens d'obturation.
La figure 5 est une vue latérale du boîtier.
La figure 6 illustre le corps de boîtier en coupe dans un plan transversal (H).
La figure 7 illustre le corps de boîtier en coupe dans le plan longitudinal de symétrie (P).
Les figures 8a, 8b, 8c et 8d illustrent l'enceinte déformable destinée à recevoir la poche souple.

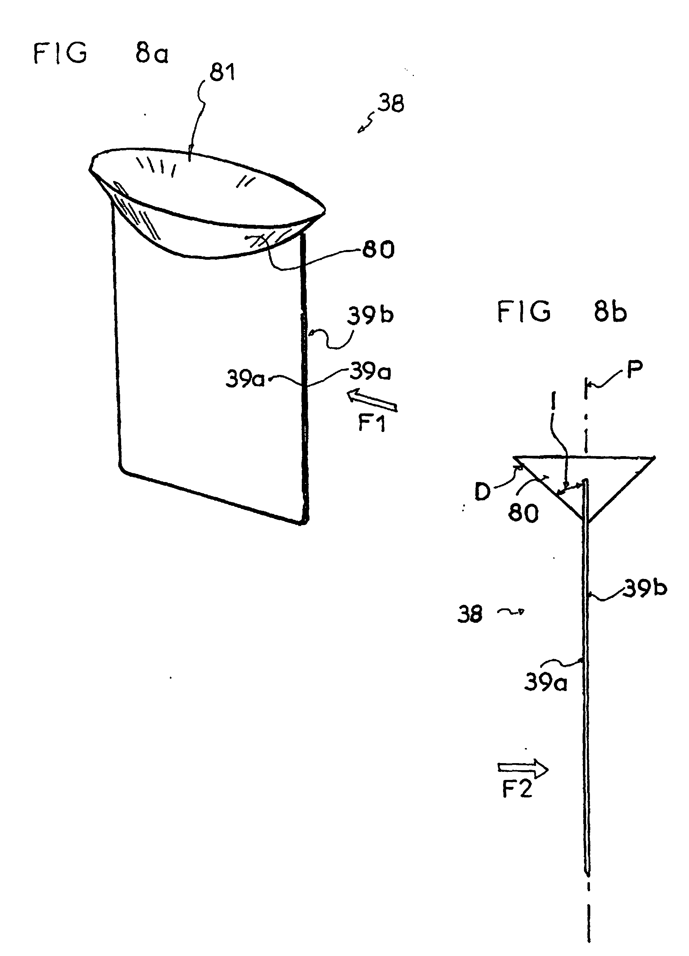

La figure 8a illustre l'enceinte déformable en perspective.
La figure 8b représente l'enceinte en vue latéral selon F1.

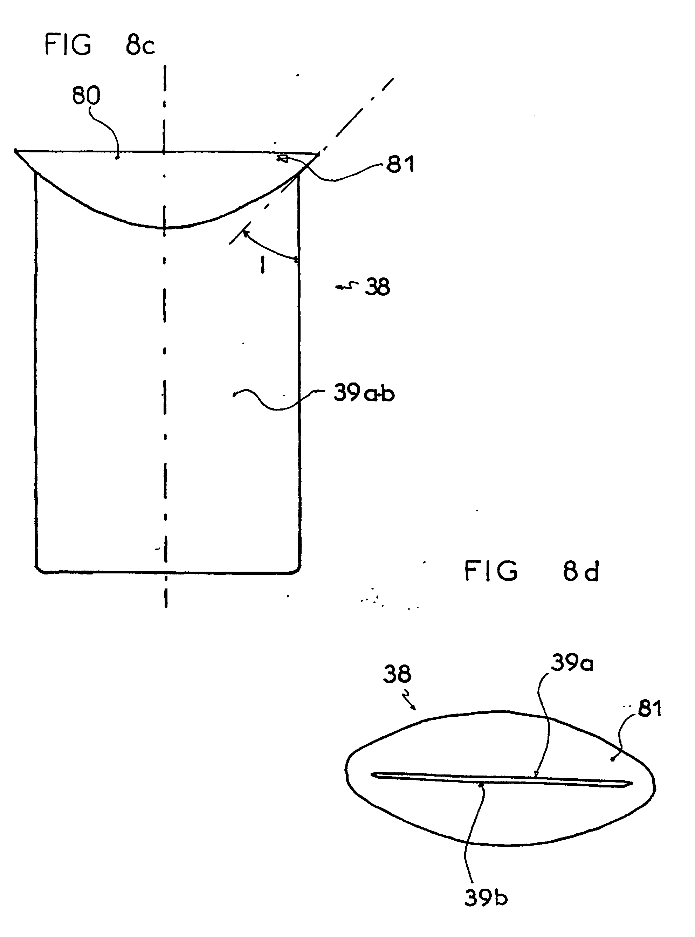

La figure 8c illustre l'enceinte en vue latérale selon F2
La figure 8d illustre l'enceinte en vue de dessus.

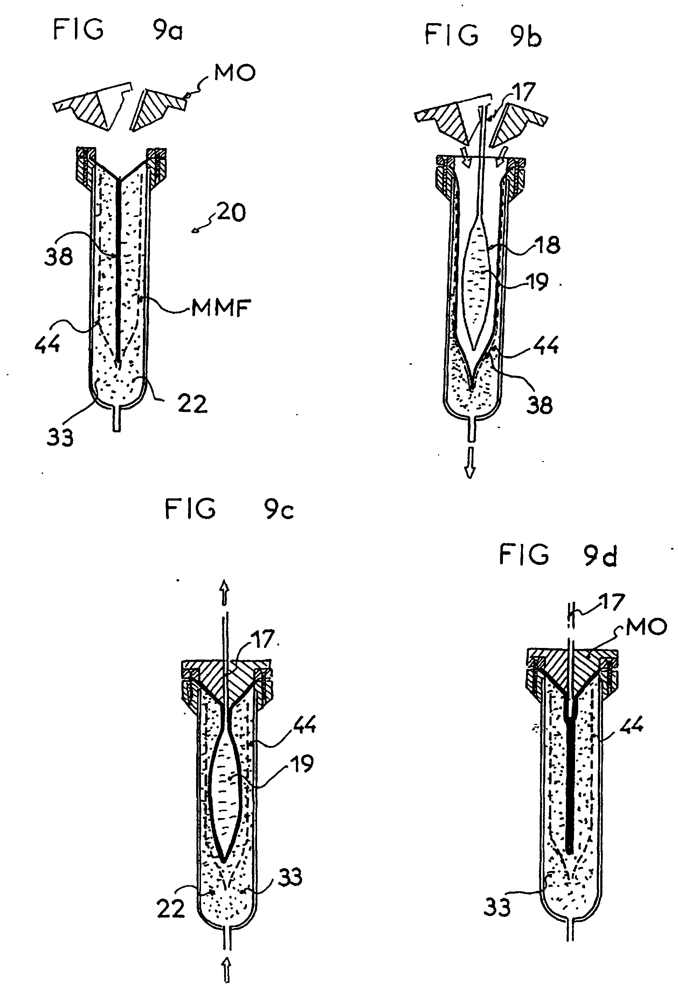

Les figures 9a, 9b, 9c et 9d illustrent l'étape d'introduction de la poche souple à l'intérieur du dispositif ainsi que l'étape d'injection, en coupe dans un plan vertical (Q).
La figure 9a illustre la position initiale.
La figure 9b illustre la dépression et la mise en forme de la membrane ainsi que l'introduction de la poche.
La figure 9c illustre l'étape d'injection.
La figure 9d illustre le dispositif à la fin de l'étape d'injection.

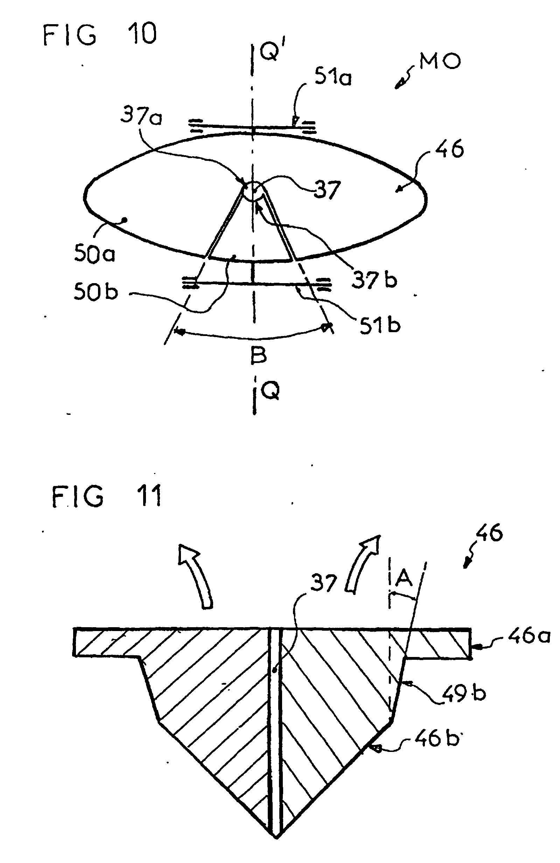

La figure 10 illustre schématiquement le couvercle ou bouchon en vue de dessus.
La figure 11 représente ledit bouchon en coupe selon (QQ')

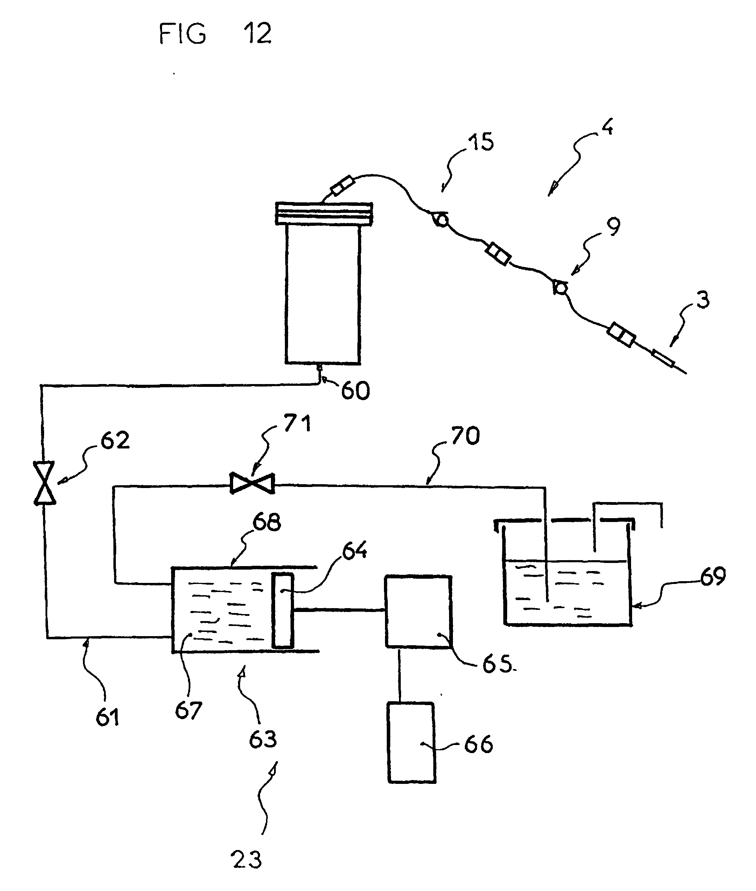

La figure 12 est un schéma de l'installation selon un mode préferé de l'invention.

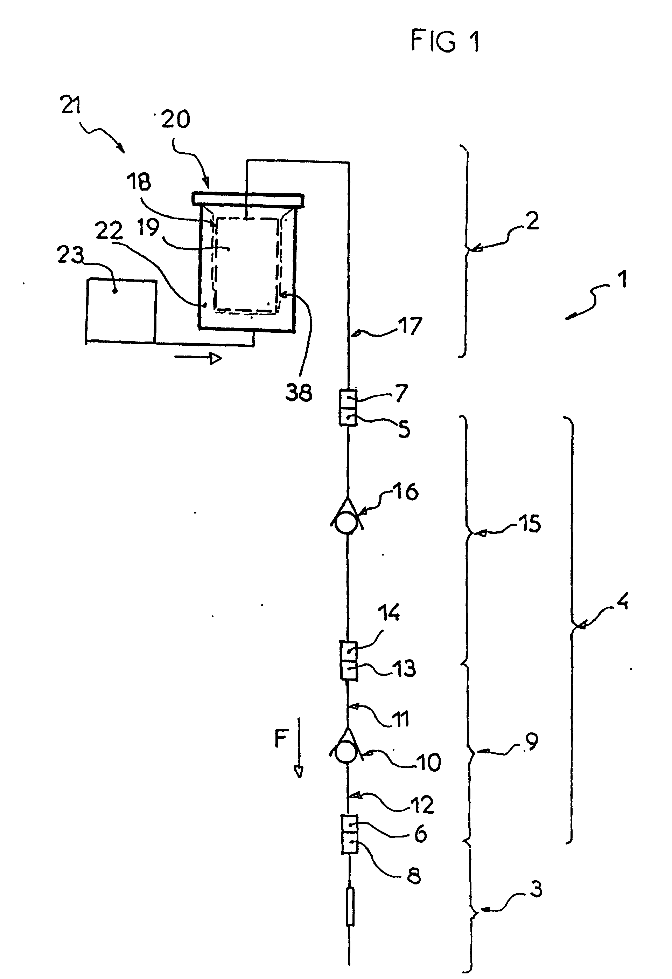

La figure 1 illustre schématiquement le dispositif d'injection selon un mode préféré de l'invention qui porte la référence globale (1). Ledit dispositif comprend un injecteur proprement dit ou dispositif d'alimentation (2) relié à un conduit d'injection (3) tel qu'un cathéter, une aiguille hypodermique ou intraveineuse par l'intermédiaire d'une canalisation (4) constituée de plusieurs éléments. Ladite canalisation (4) comprend, à ses extrémités, deux raccords de branchement, un raccord amont (5) et un raccord aval (6). Le raccord amont (5) est destiné à être raccordé à un raccord d'alimentation (7) solidaire de l'injecteur, tandis que le raccord aval (6) est destiné à être raccordé au raccord amont (8) du conduit d'injection (3). Par ailleurs et avantageusement, la canalisation (4) comprend un dispositif de sécurité (9) tel que décrit dans la demande française n° 93 12590 et qui est constitué par un raccord amovible comprenant une valve anti-retour à seuil (10) comprise entre une canalisation amont (11) et une canalisation aval (12). La valve anti-retour à seuil (10) est adaptée pour autoriser le passage du liquide d'amont en aval comme représenté par la flèche "F" lorsqu'une pression déterminée du fluide en écoulement est atteinte, tandis qu'elle condamne le passage du liquide médical en sens inverse, à savoir, d'aval en amont, c'est-à-dire dans le sens contraire de celui illustré par la flèche "F". Notons que la canalisation amont (11) du dispositif de sécurité (9) comprend un raccord amont (13) destiné à être raccordé à un raccord aval (14) d'une portion amont de la canalisation (4) qui comprend par ailleurs une autre valve anti-retour à seuil (16), tandis que la canalisation aval (12) comprend le raccord aval (6) précédemment décrit.

Ajoutons que la canalisation (4) est connectée à l'injecteur (2) par raccordement du raccord amont (5) de ladite canalisation au raccord d'alimentation (7). Selon l'une des caractéristiques de l'invention, ledit raccord (7) est directement fixé à l'extrémité du conduit d'alimentation (17) d'une poche souple (18) contenant le liquide médical (19) à injecter. Ladite poche souple (18) étant disposée dans un boîtier de mise en pression (20) d'un dispositif de mise en pression (21) de l'injecteur (2). Selon l'invention, la mise en pression de la poche souple est réalisée par un liquide moteur inerte enveloppant ladite poche. Ledit liquide moteur inerte (22) étant contenu dans le boîtier (20), et sa mise en pression étant réalisée par des moyens de mise en pression (23) tels qu'une pompe haute pression ou un vérin comprenant un piston coulissant actionné par un moteur électrique dont l'avance est contrôlée, ou tout autre dispositif motorisé voire même manuel.

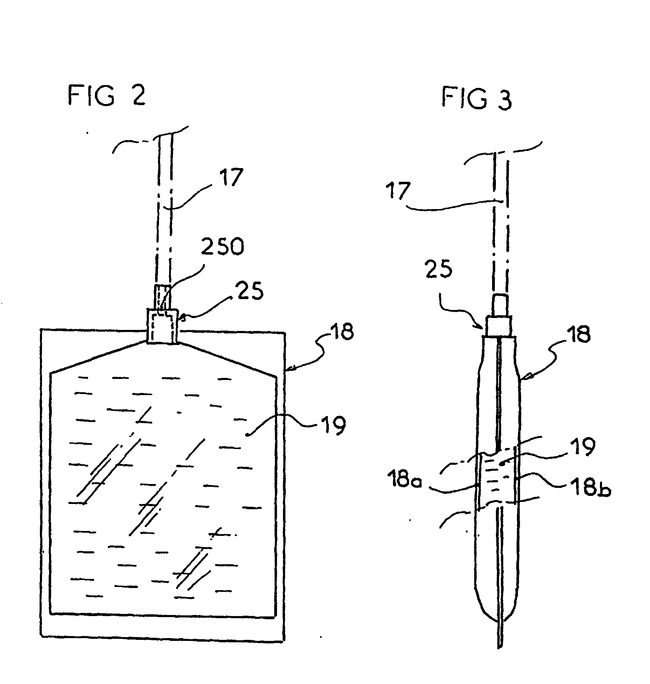

Les figures 2 et 3 illustrent schématiquement une poche (18) telle qu'utilisée par le dispositif d'injection et contenant le liquide médical (19) à injecter. Ladite poche (18) est constituée par l'assemblage de deux feuilles en matériau plastique souple, une première feuille (18a) et une deuxième feuille identique (18b) associées périphériquement par collage ou soudage de façon à constituer un volume interne (18c) destiné à contenir le liquide médical (19). Par ailleurs, la partie inférieure de la poche comprend un tube d'écoulement (25) obturé provisoirement par une membrane (250) que l'on perce lors du branchement dudit tube sur le conduit d'alimentation (17). Bien entendu, la poche (18) pourrait comprendre sont conduit d'alimentation (17) tel que représenté en traits mixtes. La poché ainsi réalisée et contenant le liquide est molle et déformable et est, selon l'invention, mise en place dans le liquide moteur (22) d'un boîtier (20), ledit liquide enveloppant la totalité de la poche et étant mis en pression pour déformer ladite poche de façon homogène et périphérique, afin de forcer le liquide médical à s'écouler dans la canalisation (4).

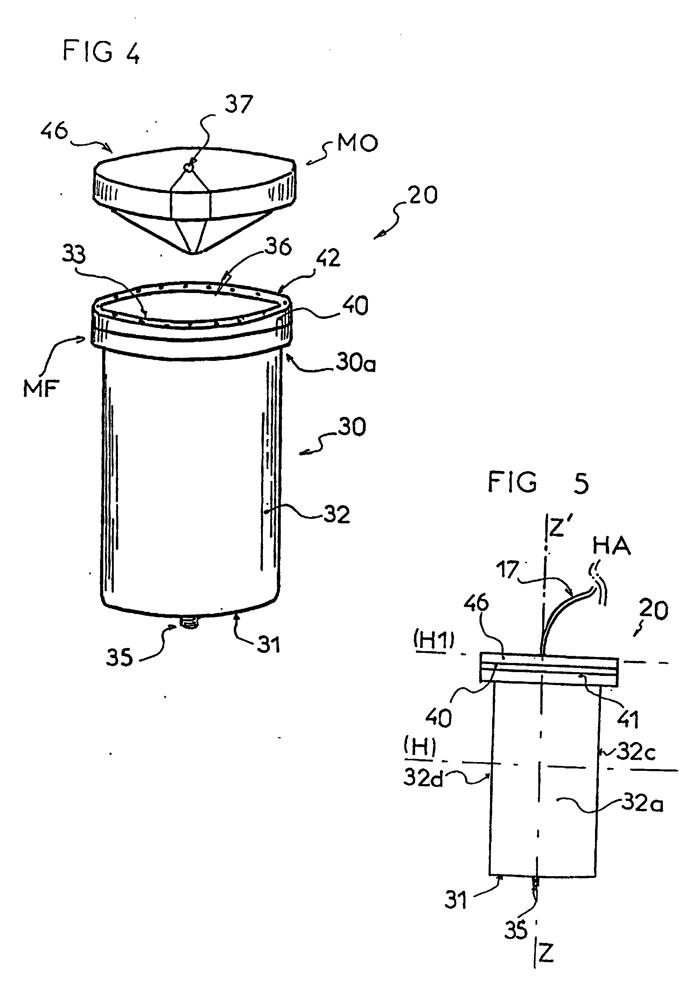

Selon le mode de réalisation préféré de l'invention, le boîtier (20) illustré figures 4 à 7, est constitué par un corps de boîtier (30) de plan longitudinal de symétrie générale (P) composé d'une paroi d'extrémité inférieure (31) et d'une paroi périphérique latérale (32) formant une enceinte interne (33) destinée à contenir le liquide moteur inerte (22). Ledit corps de boîtier (30) comporte un orifice de raccordement (34) permettant de raccorder les moyens de mise en pression (23) du dispositif d'injection (1) avec le liquide moteur (22) contenu dans l'enceinte interne (33), ledit orifice de raccordement étant avantageusement situé dans la paroi d'extrémité inférieure (31) et étant muni d'un prolongement de paroi cylindrique (35) avantageusement fileté s'étendant vers l'extérieur (EXT) afin de faciliter ledit raccordement. Bien entendu, les moyens de raccordement entre le corps de boîtier (30), plus particulièrement ledit prolongement (35), et les moyens de mise en pression (23) peuvent être de tous types connus.

Le corps de boîtier (30) comporte à son extrémité supérieure (30a) une ouverture supérieure (36) destinée à permettre l'introduction de la poche souple (18) contenant le liquide médical (19) dans l'enceinte interne (33) du boîtier (20). Il est prévu, en outre, des moyens d'obturation (MO) de l'ouverture (36) du corps de boîtier (30), lesdits moyens d'obturation (MO) étant mobiles entre une position d'ouverture destinée à permettre l'introduction de la poche souple à l'intérieur de l'enceinte (33) et une position d'obturation permettant la fermeture de ladite enceinte. Lesdits moyens d'obturations (MO) comportent avantageusement un orifice de passage (37) destiné à permettre le passage du conduit d'alimentation (17) et/ou d'une portion du tube d'écoulement (25) de manière à permettre le raccordement de la poche souple (18) à la canalisation (4) lorsque ladite poche est disposée à l'intérieur (INT) de l'enceinte interne (33) du boîtier (20), comme le montre la figure 5.

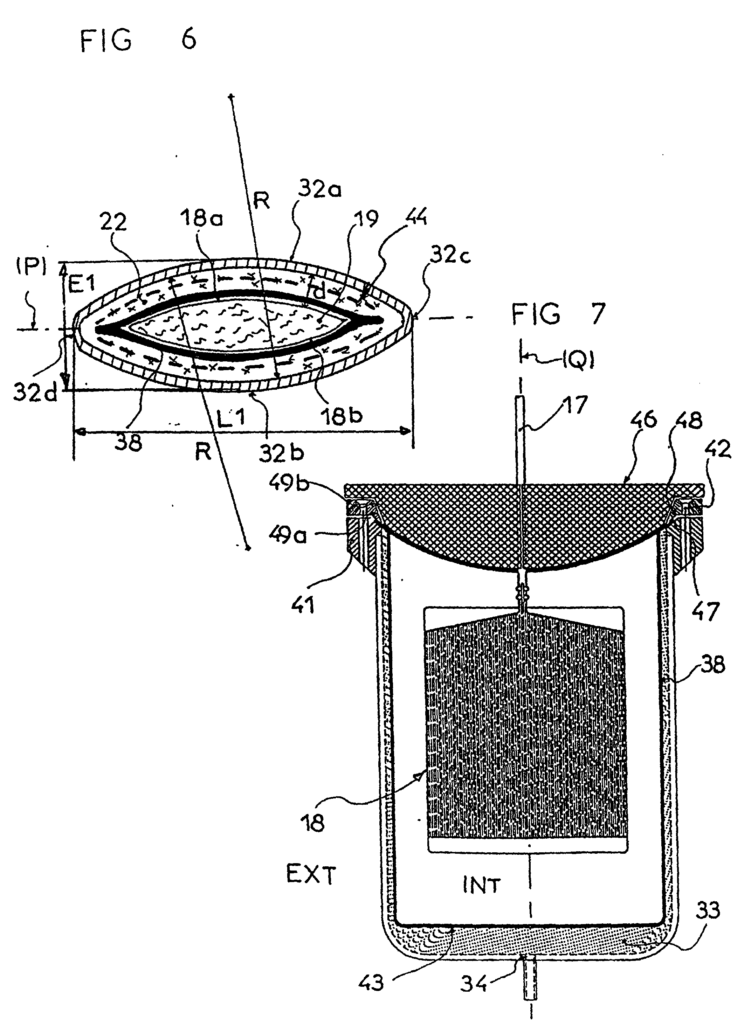

Selon l'invention, la paroi périphérique latérale (32) du corps de boîtier (30) présente, dans un plan transversal (H) perpendiculaire au plan de symétrie (P), une section transversale allongée de forme ovale ou ovoïde. Ladite paroi périphérique est constituée, selon le mode de réalisation préféré, par deux portions de paroi latérales (32a, 32b) de forme courbe, avantageusement bombées vers l'extérieur de l'enceinte, leur section respective dans le plan transversal (H) étant avantageusement constituée chacune par une portion de cercle, lesdites portions de cercles étant non concentriques et de rayon "R" identiques, tel qu'illustré figure 6. Notons que le rayon "R" est avantageusement compris entre 10 et 18 cm et peut être choisi sensiblement égal à 14 cm.

Les portions de paroi latérales (32a, 32b) sont disposées symétriquement de part et d'autre du plan longitudinal (P) et sont par exemple reliées l'une à l'autre par deux cordons de soudure longitudinaux (32c, 32d) comme le montre les figures 5 et 6, lesdits cordons de soudure pouvant constituer deux portions de paroi recourbées dont le rayon de courbure dans le plan transversal (H) est faible.

Selon le mode de réalisation préféré de l'invention, le corps de boîtier (30) possède ainsi une section transversale de forme oblongue bombée latéralement dont la forme et les dimensions sont sensiblement constantes selon l'axe longitudinal (ZZ') du boîtier. Il va de soi que les dimensions de ladite section transversale comme, par exemple, le rayon "R" des parois périphériques latérales (32a, 32b) pourraient varier longitudinalement selon l'axe (ZZ') sans pour autant sortir du champ de protection de l'invention. Ainsi, lorsqu'une poche souple (18) remplie est introduite dans l'enceinte (33), la forme ovale allongée de la section transversale du corps de boîtier (30) permet à la distance « d » entre les portions de paroi périphérique (32a, 32b) dudit corps et les parois (18a, 18b) de ladite poche d'être sensiblement constante dans un plan transversal comme le montre la figure 6.

Selon le mode de réalisation préféré de l'invention, une enceinte déformable (38) destinée à recevoir la poche souple (18) s'étend à l'intérieur (INT) de l'enceinte interne (33) depuis l'ouverture supérieure (36) située à l'extrémité supérieure (30a) du corps de boîtier (30). Ladite enceinte déformable est fixée hermétiquement sensiblement au niveau de l'ouverture supérieure (36) à l'intérieur de l'enceinte interne (33) par des moyens de fixation (MF) de manière à être disposée et à pouvoir accueillir ainsi la poche souple (18) à l'intérieur de ladite enceinte interne (33) sans que celle-ci ne soit en contact direct avec le liquide moteur interne (22).

L'enceinte déformable (38) illustrée figure 8a à 8d est avantageusement constituée par deux parois déformable souples (39a, 39b) dont les bords latéraux et inférieurs sont solidarisés hermétiquement et qui est prolongé vers le haut dans sa partie supérieure par une paroi divergente (80) dont les bords supérieurs (81) sont destinés à être fixés hermétiquement par les moyens de fixation (MF) sur la périphérie de l'ouverture supérieure (36) permettant ainsi l'introduction dans ladite enceinte déformable de la poche souple. La paroi divergente (80) est formée par une surface définie sensiblement par une portion de droite (D) formant un angle (I) sensiblement constant avec un plan vertical sensiblement égale à 45° et parcourant la courbe contenue dans le plan transversal (H) correspondant à la courbe de forme allongée ovale ou ovoïde de la paroi périphérique latérale (32) dans ledit plan situé au niveau de l'ouverture supérieur (36) tel qu'illustré figure 8d. La forme de la paroi divergente (80) peut être arrondie dans sa partie inférieure selon le plan longitudinal (P) comme le montre la figure 8c. Notons que les moyens de fixation (MF) sont avantageusement constitués par un organe de fixation complémentaire (40) destiné à coopérer avec un rebord supérieur (41) du corps de boîtier (30) faisant saillie vers l'extérieur (EXT) dudit corps dans un plan transversal (H1) situé sensiblement au niveau de l'ouverture supérieure (36) de manière à fixer par coincement les bords supérieurs (81) de la paroi divergente (80) des parois souples (39a, 39b) constituant l'enceinte déformable (38) entre ledit organe et le rebord dans le plan transversal (H1), ladite paroi divergente (80) formant ainsi un joint d'étanchéité. L'organe complémentaire (40) est fixé contre le rebord (41) à l'aide de vis (42) ou de rivets, par exemple. Notons que le coincement des parois souples peut s'effectuer le long d'un chanfrein (47) de l'ouverture (36) du corps de boîtier et une surface complémentaire (48) de l'organe de fixation complémentaire (40), ledit organe laissant libre l'ouverture (36), comme le montre la figure 7. Il va de soi que les moyens de fixation (MF) de l'enceinte déformable (38) avec le corps de boîtier (30) pourraient être obtenus par des systèmes équivalents sans pour autant sortir du champ de protection de l'invention.

Selon le mode de réalisation préféré de l'invention, l'enceinte déformable (38) est réalisée dans un matériau souple et déformable tel qu'en Latex, en Néoprène, en silicone ou en élastomère polydiméthyle.

Par ailleurs, le dispositif d'injection (1) peut comporter des moyens de positionnement non représentés de l'enceinte déformable (38) de manière à ce que ladite enceinte s'étende sensiblement dans le plan (P) afin de pouvoir maintenir la poche souple (18) dans ledit plan (P). Selon le mode de réalisation préféré, lesdits moyens de positionnement sont constitués par une tige métallique solidaire du bord inférieur (43) de l'enceinte déformable, ladite tige étant maintenue dans la plan P par des moyens de liaison rigides la reliant à la paroi d'extrémité inférieure, par exemple, ou par des moyens de liaison semi-rigides lui autorisant un faible débattement longitudinal.

En outre, afin de permettre une introduction aisée de la poche souple (18) à l'intérieur de l'enceinte déformable, le dispositif (1) comporte des moyens de mise en forme (MMF) de ladite enceinte destinés à permettre à ladite enceinte déformable (38) de prendre une forme s'approchant sensiblement de la forme d'une poche souple remplie. Lesdits moyens de mise en forme (MMF) sont constitués, par exemple, par une grille de guidage ou un grillage rigide (44) dont la forme correspond à celle d'une poche souple (18) remplie destinée à être utilisée dans le dispositif (1), ladite grille (44) étant disposée autour de l'enceinte déformable (38). Ainsi, les moyens de mise en forme (MMF) sont destinés à permettre aux parois souples (39a, 39b) formant l'enceinte déformable (38) de s'écarter l'une de l'autre de manière à venir contre les parois de la grille de guidage (44) laissant ainsi à l'utilisateur un accès facilité à l'enceinte (38). Celle-ci ayant de ce fait pris sensiblement la forme d'une poche souple remplie, l'introduction dans l'enceinte déformable (38) de la poche (19) s'effectue aisément. Il va de soi que les moyens de mise en forme (MMF) pourraient être remplacés par un système équivalent sans pour autant sortir du champ de protection de l'invention.

Selon le mode de réalisation préféré de l'invention, les moyens d'obturation (MO) de l'enceinte interne (33) sont constitués par un bouchon ou couvercle (46) amovible illustré particulièrement figures 10 et 11. Ledit couvercle (46) est constitué par un plateau supérieur (46a) dont la forme et les dimensions sont avantageusement identiques au contour de la face supérieure de l'organe de fixation complémentaire (40). Le plateau supérieur (46a) est prolongé vers le bas par un volume (46b) dont la section dans un plan transversal situé à proximité de l'ouverture supérieure (36) présente une forme allongée ovale ou ovoïde complémentaire de celle de la face interne de la paroi périphérique latérale (32) au jeu près dans ledit plan afin de pouvoir obturer hermétiquement l'enceinte interne (33).

Les dimensions de la section transversale du bouchon (46) diminuent progressivement dans la partie inférieure dudit volume (46b) de manière à épouser la forme évasée de la paroi divergente (80) prolongeant les parois souples (39a, 39b) de l'enceinte déformable (38). Ainsi selon le mode de réalisation préféré de l'invention, la partie inférieure du bouchon (46b) possède sensiblement une forme constituée par le parcours le long de la courbe de forme allongée ovale ou ovoïde formant la section transversale de la paroi périphérique latérale dans un plan horizontal situé au niveau de l'ouverture supérieure (36), d'une portion de droite orientée sensiblement à 45°. Notons que selon le mode de réalisation préféré de l'invention, l'ouverture supérieure (36) et le volume (46b) du bouchon peuvent coopérer grâce à un chanfrein d'entrée (49a) de l'ouverture supérieure (36) et à un chanfrein complémentaire (49b) du bouchon dont l'inclinaison A peut avantageusement être choisie sensiblement égale à 15°, tel qu'illustré figure 11. De plus, la forme évasée de la partie inférieure (46b) du bouchon est destinée à coopérer de façon étroite et complémentaire avec la forme particulière de la paroi divergente (80) de l'enceinte déformable. Ce type de disposition permet une parfaite compatibilité entre les manoeuvres d'ouverture et de fermeture.

Par ailleurs, l'orifice de passage (37) des moyens d'obturation (MO) est avantageusement constitué par un canal de passage situé au centre du bouchon (46) selon l'axe longitudinal (ZZ'). Selon le mode de réalisation préféré, ledit bouchon est constitué en deux parties (50a, 50b) complémentaires, lesdites parties étant réalisées de manière à ce que le canal de passage (37) soit constitué par deux gorges (37a, 37b) complémentaires situées chacune sur une des parties du bouchon (50a, 50b). De ce fait, lesdites parties sont réalisées de manière à permettre le placement du conduit d'alimentation (17) et/ou d'une portion du tube d'écoulement (25) dans un des gorges (37a, 37b) lorsque les deux parties (50a, 50b) sont séparées. Notons que la partie (50b) est un secteur centré sur l'axe (ZZ') d'angle B compris entre 10° et 90°, par exemple. Les deux parties (50a, 50b) sont avantageusement articulées chacune en pivotement par rapport au corps de boîtier (30) par deux articulations de type charnière (51a, 51b) illustrées schématiquement figure 10, entre la position d'ouverture du bouchon (46) et la position d'obturation de l'enceinte. Des moyens de verrouillage non illustrés du couvercle (46) sur le corps de boîtier (30) sont prévus afin de pouvoir permettre la mise en pression du liquide moteur inerte (22) à l'intérieur de l'enceinte (33), lorsque ledit couvercle obture hermétiquement l'ouverture (36) de l'enceinte (33). Notons, par ailleurs, que le corps de boîtier (30) peut avantageusement être réalisé en aluminium ou en acier, par exemple, de manière à pouvoir mettre le liquide moteur en pression maximale comprise entre 80 à 100 bars, le liquide moteur inerte (22) peut, par exemple, être de l'eau, de la glycérine ou un mélange des deux.

De plus, afin de faciliter l'enlèvement des bulles d'air indésirables que pourrait contenir la poche souple (18), le boîtier (20) est orienté de manière à ce que l'orifice d'écoulement de ladite poche reliée à la canalisation débouche selon l'axe longitudinal (ZZ') vers le haut (HA), l'ouverture supérieure (36) du corps de boîtier (30) étant située à l'extrémité supérieure dudit corps.

L'enceinte (33) est alimentée en liquide moteur (22) par un conduit d'arrivée (60) connecté à une tubulure (61), ladite tubulure comprenant une vanne principale électrique (62) reliée à un vérin (63) dont le piston (64) est commandé en déplacement par un moteur électrique (65) dont l'avance est contrôlée par un automatisme (66).

Par ailleurs, la chambre (67) du cylindre (68) du vérin (63) est reliée à un réservoir supplémentaire de liquide moteur (69) par un conduit secondaire (70) et peut comprendre une vanne secondaire électrique (71), comme le montre la figure 12.

Ainsi, dans une phase préliminaire d'utilisation du dispositif pendant laquelle est faite la remise à zéro, il est prévu de fermer l'enceinte (33) par les moyens d'obturation (MO) sans y avoir mis en place une poche de liquide à injecter, les deux vannes (62, 71) étant ouvertes, le piston (64) étant ensuite reculé, la vanne (71) étant alors fermée, l'affichage du zéro étant alors fait par avance du piston.

Le procédé d'injection comporte ensuite une étape d'introduction de la poche souple dans le dispositif formé par le boîtier (20) comme le montre les figures 9a et 9b. Lors de cette étape, le dispositif de mise en pression (21) provoque en retirant de l'enceinte (33) une partie du liquide moteur (22) qu'elle contient, une dépression dans ladite enceinte. De ce fait, les parois souples (39a, 39b) de l'enceinte déformable prennent la forme d'une poche souple remplie grâce aux moyens de mise en forme (MMF) constitués par la grille de guidage (44). La poche souple (18) est alors introduite dans l'enceinte déformable (38) en prenant soin de positionner ou de faire passer le canal d'alimentation (17) dans l'orifice de passage (37) des moyens d'obturation (MO) comme le montre la figure 9b, lesdits moyens d'obturation étant alors positionnés en position d'obturation..

L'étape d'injection proprement dite illustrée figure 9c consiste grâce aux moyens de mise en pression (23) à réintroduire du liquide moteur (22) à l'intérieur de l'enceinte (33) afin d'obtenir une pression choisie à l'intérieur de ladite enceinte de manière à provoquer la déformation de la poche souple (18). Cette déformation provoque ainsi l'écoulement du liquide médical (19) dans la canalisation (4) par l'intermédiaire du canal d'alimentation (17). Lorsque la poche souple est vide comme le montre la figure 9d, l'injection est terminée et la mise en pression du liquide moteur à l'intérieur de l'enceinte (33) peut être stoppée afin de permettre l'ouverture des moyens d'obturation (MO) pour remplacer la poche souple vide par une poche souple pleine en recommençant le processus précédemment décrit.

Il va de soi que l'on ne sortira pas du cadre de l'invention quel que soit les types de moyens de mise en pression utilisés car ceux-ci peuvent être, par exemple, aussi bien motorisés que manuels.

De même, la gestion des différentes vannes éventuelles ou organes destinés à gérer les débits, peut être automatique, semi-automatique ou manuelle. Notons qu'avantageusement les variations de volume et de débit sont constamment contrôlées afin d'assurer une injection précise et sûre.

On pourrait aussi prévoir un système de sécurité qui empêche toute injection dès que le boîtier est déverrouillé. Par ailleurs, il va de soi que le dispositif comprend un support qui peut être mobile ou fixe et qui peut être en appui sur le sol ou suspendu sur un portique ou encore fixé au plafond ou à un mur, ledit support pouvant comprendre aussi un tableau de commande et de surveillance.

Il va de soit que le corps du boîtier pourrait avoir toute forme de section transversale ovale ou ovoide allongée de telle sorte que la largeur (L1) soit supérieur à l'épaisseur (E1). Notons que le boîtier peut être réalisé en toute matière comme, par exemple, en acier tel qu'inoxydable, en aluminium, ou en matière plastique. Ajoutons que ledit boîtier peut être réalisé à partir d'un tube cylindrique que l'on aplatirait par déformation.

Bien entendu, il peut être prévu des moyens retenant la partie basse de l'enceinte déformable de façon à lui interdire de remonter intempestivement.

Par ailleurs, la taille du boîtier pourrait être différente, de manière à pouvoir contenir plusieurs poches de liquide médical à injecter qui seraient accolées l'une à l'autre dans l'enceinte. Notons également que les matériaux utilisés pour réaliser les éléments constitutifs du dispositif d'injection tel que le boîtier, par exemple, peuvent avantageusement présenter un caractère amagnétique de manière à ne pas perturber des examens du type de ceux utilisant la résonance magnétique.

## Revendications

1. Dispositif destiné à mettre en oeuvre un procédé d'injection de liquide à usage médical du type qui consiste
- à utiliser une poche souple (18) contenant ledit liquide médical (19) à injecter, ladite poche étant étanche et comprenant au moins un orifice d'écoulement destiné à être connecté à une canalisation (4) reliée à un conduit d'injection (3) tel qu'un cathéter ou une aiguille hypodermique, intraveineuse ou autre,
- à déformer la poche souple (18) par l'intermédiaire d'un liquide moteur inerte (19) mis en pression de façon à transmettre cette pression au liquide médical à injecter afin de la forcer à s'écouler dans la canalisation (4) en la plaçant dans l'enceinte interne (33), d'un boîtier (20) comprenant le liquide moteur inerte (22), puis à mettre en pression ledit liquide moteur,
le dispositif comportant un boîtier (20) de plan de symétrie générale longitudinal (P) étant constitué par un corps de boîtier (30) comportant une ouverture supérieure (36) située à l'extrémité supérieure (30a) dudit corps et des moyens d'obturation (MO) mobiles entre une position d'ouverture destinée à permettre l'introduction de la poche souple (18)et une position d'obturation fermant l'enceinte,
**caractérisé en ce que** le boîtier (20) comprend une enceinte déformable (38) destinée à recevoir la poche souple (18) s'étendant à l'intérieur (INT) de l'enceinte interne (33) depuis l'ouverture supérieure (36) située à l'extrémité supérieure (30a) du corps de boîtier (30), tandis-que ladite enceinte est constituée par deux parois déformable souples (39a, 39b) dont les bords latéraux et inférieurs sont solidarisés hermétiquement ledit corps de boîtier possédant une section transversale de forme allongée ovale ou ovoïde.

2. Dispositif selon la revendication 1, **caractérisé en ce que** ladite enceinte déformable, est fixée hermétiquement dans le boîtier, sensiblement au niveau de l'ouverture supérieure (36) à l'intérieur de l'enceinte interne (33) par des moyens de fixation (MF) de manière à être disposée et à pouvoir accueillir ainsi la poche souple (18) à l'intérieur de ladite enceinte interne (33) sans que celle-ci ne soit en contact direct avec le liquide moteur interne (22).

3. Dispositif selon la revendication 2, **caractérisé en ce que** ladite enceinte déformable est prolongé vers le haut dans sa partie supérieure par une paroi divergente (80) dont les bords supérieurs (81) sont destinés à être fixés hermétiquement par les moyens de fixation (MF) sur la périphérie de l'ouverture supérieure (36) permettant ainsi l'introduction dans ladite enceinte déformable de la poche souple.

4. Dispositif selon la revendication 1, **caractérisé en ce que** le rayon "R" de la section transversale des portions de paroi latérales (32a, 32b) est constant le long de l'axe longitudinal (ZZ') du corps de boîtier (30).

5. Dispositif selon l'une quelconque des revendications précédentes, **caractérisé en ce qu'**il comporte des moyens de positionnement destinés à permettre le positionnement de l'enceinte déformable (38) sensiblement dans le plan de symétrie longitudinale (P).

6. Dispositif selon l'une quelconque des revendications précédentes, **caractérisé en ce qu'**il comporte des moyens de mise en forme (MMF) de l'enceinte déformable (38) destinés à permettre à ladite enceinte de prendre sensiblement la forme d'une poche souple remplie avant l'introduction de la poche souple (18).

7. Dispositif selon l'une quelconque des revendications précédentes, **caractérisé en ce que** les moyens d'obturation (MO) comportent un orifice de passage (37) permettant de raccorder la poche souple (18) à la canalisation (4).

8. Dispositif selon l'une quelconque des revendications précédentes, **caractérisé en ce que** les moyens d'obturation (MO) sont formés par un bouchon (46)constitué en deux parties complémentaires (50a, 50b) mobiles l'une par rapport à l'autre.

9. Dispositif selon l'une quelconque des revendications précédentes, **caractérisé en ce que** les moyens d'obturation (MO) sont formés par un bouchon (46) ou couvercle (46) amovible dont lasection dans un plan transversal situé à proximité de l'ouverture supérieure (36) présente une forme allongée ovale ou ovoïde complémentaire de celle de la face interne de la paroi périphérique latérale (32) au jeu près dans ledit plan afin de pouvoir obturer hermétiquement l'enceinte interne (33).

10. Dispositif selon l'une quelconque des revendications précédentes, **caractérisé en ce que** le plan de symétrie générale longitudinal (P) du boîtier (20) est disposé sensiblement verticalement, l'ouverture supérieure (36) du corps de boîtier (30) étant dirigée vers le haut (HA).

11. Dispositif selon l'une quelconque des revendications précédentes, **caractérisé en ce que**, la canalisation (4) comprend un dispositif de sécurité (9) comprenant une valve anti-retour à seuil (10) qui est adaptée pour autoriser le passage du liquide d'amont en aval lorsqu'une pression déterminée du fluide en écoulement est atteinte, tandis qu'elle condamne le passage du liquide médical en sens inverse, à savoir, d'aval en amont

## Claims

1. Device intended to implement a method of injecting liquid for medical usage of the type which consists of
- using a flexible pouch (18) containing the said medical liquid (19) to be injected, the said pouch being liquidtight and comprising at least one drainage orifice intended to be connected to a pipe (4) connected to an injection conduit (3) such as a catheter or a hypodermic needle, intravenous or other,
- deforming the flexible pouch (18) by means of an inert drive liquid (19) pressurised so as to transmit this pressure to the medical liquid to be injected in order to force it to flow through the pipe (4) by placing it in the internal enclosure (33) of a casing (20) including the inert drive liquid (22), then pressurising the said drive liquid, the device comprising a casing (20) with a longitudinal overall plane of symmetry (P) consisting of a casing body (30) having a top opening (36) situated at the top end (30a) of the said body and closure means (MO) able to move between an open position intended to allow the introduction of the flexible pouch (18) and a closed position closing off the enclosure,
**characterised in that** the casing (20) comprises a deformable enclosure (28) intended to receive the flexible pouch (18) extending inside (INT) the internal enclosure (33) from the top opening (36) situated at the top end (30a) of the casing body (30), whilst the said enclosure is formed by two flexible deformable walls (39a, 39b) whose lateral and bottom edges are hermetically connected, the said casing body having a transverse section with an oval or ovoid elongate shape.

2. Device according to Claim 1, **characterised in that** the said deformable enclosure is fixed hermetically in the casing, substantially at the level of the top opening (36) inside the internal enclosure (33) by fixing means (MF) so as to be disposed and to be able thus to accept the flexible pouch (18) inside the said internal enclosure (33) without its being in direct contact with the internal drive liquid (22).

3. Device according to Claim 2, **characterised in that** the said deformable enclosure is extended upwards in its top part by a divergent wall (80) whose top edges (81) are intended to be hermetically fixed by the fixing means (MF) on the periphery of the top opening (36), thus enabling the flexible pouch to be introduced into the said deformable enclosure.

4. Device according to Claim 1, **characterised in that** the radius (R) of the transverse section of the lateral wall portions (32a, 32b) is constant along the longitudinal axis (ZZ') of the casing body (30).

5. Device according to any one of the preceding claims, **characterised in that** it has positioning means intended to allow the positioning of the deformable enclosure (38) substantially in the longitudinal plane of symmetry (P).

6. Device according to any one of the preceding claims, **characterised in that** it has means (MMF) of shaping the deformable enclosure (38) intended to enable the said enclosure to take substantially the shape of a filled flexible pouch before the introduction of the flexible pouch (18).

7. Device according to any one of the preceding claims, **characterised in that** the closure means (MO) have a passage orifice (37) for connecting the flexible pouch (18) to the pipe (4).

8. Device according to any one of the preceding claims, **characterised in that** the closure means (MO) are formed by a plug (46) formed in two complementary parts (50a, 50b) able to move with respect to one another.

9. Device according to any one of the preceding claims, **characterised in that** the closure means (MO) are formed by a removable plug (46) or cover (46) whose cross-section in a transverse plane situated close to the top opening (36) has an oval or ovoid elongate shape complementary to that of the internal face of the lateral peripheral wall (32) except for any clearance in the said plane in order to be able to hermetically close the internal enclosure (33).

10. Device according to any one of the preceding claims, **characterised in that** the overall longitudinal plane of symmetry (P) of the casing (20) is disposed substantially vertically, the top opening (36) of the casing body (30) being directed upwards (HA).

11. Device according to any one of the preceding claims, **characterised in that** the pipe (4) comprises a safety device (9) comprising a non-return valve (10) with threshold which is adapted to allow the passage of the liquid from upstream to downstream when a given pressure of the flowing fluid is reached, whilst it blocks the passage of the medical liquid in the reverse direction, namely from downstream to upstream.

## Patentansprüche

1. Vorrichtung zur Durchführung eines Injektionsverfahrens einer medizinischen Flüssigkeit, das darin besteht
- einen flexiblen Beutel (18) zu verwenden, der die zu injizierende medizinische Flüssigkeit (19) enthält, wobei der Beutel dicht ist und mindestens eine Abflussöffnung aufweist, die an ein mit einem Injektionsrohr (3), beispielsweise einem Katheter oder einer hypodermischen, einer intravenösen oder einer anderen Nadel, verbundenen Leitungssystem (4) anschließbar ist,
- den flexiblen Beutel (18) über eine unter Druck gesetzte inerte Antriebsflüssigkeit (22) so zu verformen, dass der Druck auf die zu injizierende medizinische Flüssigkeit übertragen wird, wodurch diese dazu gezwungen wird, in das Leitungssystem (4) zu fließen, in dem er in die Innenkammer (33) eines die inerte Antriebsflüssigkeit (22) enthaltenden Gehäuses (20) eingesetzt wird, diese Antriebsflüssigkeit dann mit Druck beaufschlagt wird, wobei die Vorrichtung ein Gehäuse (20) mit einer Längssymmetrieebene (P) umfasst, das aus einem Gehäusekörper (30) besteht, welcher eine am oberen Ende (30a) des Körpers vorgesehene obere Öffnung (36) sowie Verschlussmittel (MO) umfasst, die zwischen einer Öffnungsposition zum Einführen des flexiblen Beutels (18) und einer Verschlussposition zum Schließen der Kammer beweglich sind,
**dadurch gekennzeichnet, dass** das Gehäuse (20) eine verformbare Kammer (38) umfasst, in welcher der flexible Beutel (18), der sich von der oberen, am oberen Ende (30a) des Gehäusekörpers (30) vorgesehenen Öffnung in das Innere (INT) der Innenkammer (33) erstreckt, aufnehmbar ist, während die Kammer aus zwei flexiblen, verformbaren Wandungen (39a, 39b) besteht, deren seitliche und untere Ränder hermetisch miteinander verbunden sind, wobei der Gehäusekörper einen länglichen, ovalen oder ovoiden Querschnitt aufweist.

2. Vorrichtung nach Anspruch 1, **dadurch gekennzeichnet, dass** die verformbare Kammer hermetisch im Gehäuse, im Wesentlichen auf der Höhe der oberen Öffnung (36) innerhalb der Innenkammer (33) durch Befestigungsmittel (MF) derart befestigt und angeordnet ist, dass sie den flexiblen Beutel (18) innerhalb der Innenkammer (33) aufnehmen kann, ohne dass dieser in direktem Kontakt mit der inerten Antriebsflüssigkeit (22) steht.

3. Vorrichtung nach Anspruch 2, **dadurch gekennzeichnet, dass** die verformbare Kammer in ihrem oberen Teil nach oben hin durch eine schräg sich öffnende Wandung (80) verlängert ist, deren oberen Ränder (81) durch die Befestigungsmittel (MF) hermetisch auf dem Umfang der oberen Öffnung (36) befestigbar sind, wodurch die Einführung des flexiblen Beutels in die verformbare Kammer ermöglicht wird.

4. Vorrichtung nach Anspruch 1, **dadurch gekennzeichnet, dass** der Querschnittradius "R" der Abschnitte der seitlichen Wandungen (32a, 32b) entlang der Längsachse (ZZ') des Gehäusekörpers (30) konstant bleibt.

5. Vorrichtung nach einem der vorstehenden Ansprüche, **dadurch gekennzeichnet, dass** sie Positionierungsmittel umfasst, mit denen die verformbare Kammer (38) im Wesentlichen in der Längssymmetrieebene (P) positioniert werden kann.

6. Vorrichtung nach einem der vorstehenden Ansprüche, **dadurch gekennzeichnet, dass** sie Mittel zur Formgebung (MMF) der verformbaren Kammer (38) umfasst, die es der Kammer ermöglichen, im Wesentlichen die Form eines flexiblen, gefüllten Beutels anzunehmen, bevor der flexible Beutel (18) eingerührt wird.

7. Vorrichtung nach einem der vorstehenden Ansprüche, **dadurch gekennzeichnet, dass** die Verschlussmittel (MO) eine Durchgangsöffnung (37) umfassen, über die der flexible Beutel (18) an das Leitungssystem (4) angeschlossen werden kann.

8. Vorrichtung nach einem der vorstehenden Ansprüche, **dadurch gekennzeichnet, dass** die Verschlussmittel (MO) durch einen Stöpsel (46) gebildet werden, der aus zwei sich ergänzenden Teilen (50a, 50b) besteht, die relativ zueinander beweglich sind.

9. Vorrichtung nach einem der vorstehenden Ansprüche, **dadurch gekennzeichnet, dass** die Verschlussmittel (MO) durch einen abnehmbaren Stöpsel (46) oder Deckel (46) gebildet sind, deren Querschnitt in einer in der Nähe der oberen Öffnung (36) liegenden Transversalebene eine längliche ovale oder ovoide Form aufweist, die diejenige der Innenseite der seitlichen umfänglichen Wandung (32) bis auf das Spiel in dieser Ebene derart ergänzt, dass die Innenkammer (33) hermetisch verschlossen werden kann.

10. Vorrichtung nach einem der vorstehenden Ansprüche, **dadurch gekennzeichnet, dass** die Längssymmetrieebene (P) des Gehäuses (20) im Wesentlichen senkrecht angeordnet ist, wobei die obere Öffnung (36) des Gehäusekörpers (30) nach oben hin (HA) verläuft.

11. Vorrichtung nach einem der vorstehenden Ansprüche, **dadurch gekennzeichnet, dass** das Leitungssystem (4) eine Sicherheitsvorrichtung (9) umfasst, die ein Schwellwert-Rückschlagventil (10) aufweist, das den Durchfluss der Flüssigkeit von oben nach unten freigibt, wenn ein vorgegebener Druck der fließenden Flüssigkeit erreicht ist, während es den Durchfluss der medizinischen Flüssigkeit in umgekehrter Richtung, d.h. von unten nach oben, versperrt.
